# EUROPEAN PATENT APPLICATION

(11) **EP 2 333 108 A1**
(43) Date of publication of application: **15.06.2011**
(21) Application number: 09810022.5
(22) Date of filing: 28.08.2009
(51) Int. Cl.: C12Q 1/68, C12N 15/09, G01N 33/15, G01N 33/50

(54) **METHOD FOR DETECTING THE DRUG EFFECTS OF DNA METHYLATION-INHIBITORS**

(30) Priority: 29.08.2008 JP 2008222223
(71) Applicant: School Juridical Person The Kitasato Institute, Tokyo 108-8641 (JP)
(72) Inventor: TAKAHASCHI, Shinichiro, Sagamihara-shi Kanagawa 228-8555 (JP)
(74) Representative: Strehl, Peter
(86) International application number: PCT/JP2009/065041
(87) International publication number: WO 2010/024374

(57) **Abstract**

Disclosed is a method for detecting the drug effects of DNAmethylation inhibitors that detects the drug sensitivity of gene-expressing cells to DNA methylation inhibitors based on gene expression of a transcription factor (PU. 1) and/or gene expression of metallothionein (MT) that participates in differentiation to neutrophil and monocyte lines or based on DNA methylation in the metallothionein (MT) gene promotor region. Hematopoietic tumors can be treated effectively and specifically after predicting the effects of DNA methylation inhibitors on the hematopoietic tumor cells of myelodysplastic syndrome, acute myelocytic leukemia (AML), and other hematopoietic organ diseases, so therapeutic results are improved.

## Description

### Technical Field

The present invention relates to a detection method for a drug effect of a DNA methylation inhibitor serving as a molecular target drug for hematological malignancies. More specifically, the present invention relates to a detection method for drug sensitivity to a DNA methylation inhibitor based on the expression of a transcription factor (PU.1) involved in neutrophil and monocyte lineage commitment or its target gene metallothionein (MT) or based on the methylation of an MT gene promotor region.

### Background Art

Conventional treatments of hematological malignancies with an chemotherapeutic agent alone do not yield improved therapeutic results beyond a certain level. Therefore, there is a demand for the development of a tumor-specific molecular target drug excluding the chemotherapeutic agent. Intracellular aberrant DNA methylation is estimated to be partly responsible for the pathology of hematological malignancies. Thus, a DNA methylation inhibitor is a drug that is expected to find wide applications in the future.

However, the DNA methylation inhibitor does not provide any sufficient effect when being used alone for acute myeloid leukemia (AML) (see Patent Document 1: JP 2004-529104 A (US 6613753) and Patent Document 2: JP 2006-508119 A (EP 1575582)). In view of the foregoing, there is a demand for the development of a marker for predicting drug sensitivity to such DNA methylation inhibitor.

A partial decrease in expression of a transcription factor (PU.1) involved in neutrophil and monocyte lineage commitment causes acute myeloid leukemia (AML) (Non Patent Document 1: 2004, Nat. Gen. p624-30). In order to elucidate the pathogenic mechanism of AML, the inventor of the present invention decreased the expression of PU.1 with small interfering RNAs (siRNAs) to carry out analysis using PU.1-knockdown cells (K562 PU.1 KD cells). As a result, the inventor found that the expression of a metallothionein (MT) gene involved in cell growth and drug resistance was increased, and reported that there was a negative correlation between PU.1 and MT (R=-0.43, p=0.0058) in 42 AML cases (published in The 68th Annual Meeting of the Japanese Society of Hematology 2006 (registration number: PS-2-30)).

[Patent Document 1] JP 2004-529104 A (US 6613753)
[Patent Document 2] JP 2006-508119 A (EP 1575582)
[Non-Patent Document 1] 2004, Nat. Gen. p624-30
[Non-Patent Document 2] Proceedings of the 68th Annual Meeting of the Japanese Society of Hematology 2006 (registration number: PS-2-30) (published on August 30, 2006)

### Disclosure of Invention

### Technical Problem

An object of the present invention is to provide a novel and useful detection method for a drug effect of a DNA methylation inhibitor, which serves as a marker for predicting drug sensitivity to the DNA methylation inhibitor.

### Means to Solve the Problem

In this study, the inventor of the present invention analyzed a metallothionein (MT) gene promotor mechanism. For analysis, the inventor firstly established K562 PU.1 OE cell lines as cell lines that constitutively overexpress a transcription factor (PU.1) involved in neutrophil or monocyte lineage commitment. As a result, the inventor found that the expression of MT was decreased in inverse proportion to an increase in expression of PU.1, demonstrating that the MT gene was a bona fide target gene of PU.1.

Chromatin immunoprecipitation assays clarified that the acetylation of histone H3 in an MT promoter region was enhanced in K562 PU.1 KD cells. As the region has a structure rich in CpG sequence, CpG methylation in the region up to 427 bp upstream of the transcription start site was analyzed by a bisulfite DNA sequencing method. As a result, the degree of methylation was about 40% in control cell lines, while it was decreased to about 12 to 16% in the K562 PU. 1 KD cells. In contrast, the degree of methylation was increased to about 60% in the K562 PU.1 OE cells. The results revealed that the methylation was important for the regulation.

The results also revealed that the K562 PU.1 KD cells, in which the recruitment of DNA methyl transferase (Dnmt) was presumably suppressed, were resistant to 5-azacytidine and 5-aza-2'-deoxycytidine serving as Dnmt inhibitors (DNA methylation inhibitors). The above-mentioned results revealed that the decrease in expression of PU.1 caused aberrant epigenetic regulation, and found that in applying the DNA methylation inhibitor to hematological malignant cells with myelodysplasia syndrome, AML, and any other hematopoietic diseases, studies on the expression of PU.1 or MT and the methylation ratio of its promoter region were useful for the prediction and detection of therapeutic effects.

That is, the present invention provides such a detection method for a drug effect of a DNA methylation inhibitor as described below.
1. A detectionmethod for a drug effect of a DNAmethylation inhibitor, including detecting, based on the gene expression of a transcription factor (PU.1) involved in neutrophil or monocyte lineage commitment and/or the gene expression of metallothionein (MT), the drug sensitivity of a gene-expressing cell to the DNA methylation inhibitor.
2. A detection method for a drug effect of a DNAmethylation inhibitor, the method including detecting, based on DNA methylation in a metallothionein (MT) gene promotor region, the drug sensitivity of a gene-expressing cell to the DNA methylation inhibitor.
3. The detection method for a drug effect of a DNA methylation inhibitor according to 1 above, in which the drug sensitivity of the gene-expressing cell to the DNAmethylation inhibitor is detected based on the fact that the gene expression of a transcription factor (PU. 1) involved in neutrophil or monocyte lineage commitment and/or the gene expression of metallothionein (MT) are/is regulated by the DNA methylation of a metallothionein (MT) gene promotor region in the gene-expressing cell.
4. The detection method for a drug effect of a DNA methylation inhibitor according to 1 or 3 above, in which the gene-expressing cell is detected to be resistant to the DNA methylation inhibitor based on a decrease in the gene expression of PU. 1 and/or an increase in the gene expression of MT.
5. The detection method for a drug effect of a DNA methylation inhibitor according to 1 or 3 above, in which the gene-expressing cell is detected to be highly sensitive to the DNA methylation inhibitor based on an increase in the gene expression of PU.1 and/or a decrease in the gene expression of MT.
6. The detection method for a drug effect of a DNA methylation inhibitor according to 4 above, in which the gene-expressing cell is detected to be resistant to the DNA methylation inhibitor based on the decrease in the gene expression of PU.1 and/or the increase in the gene expression of MT induced by the DNA demethylation of the MT gene promotor region in the gene-expressing cell.
7. The detection method for a drug effect of a DNA methylation inhibitor according to 5 above, in which the gene-expressing cell is detected to be highly sensitive to the DNA methylation inhibitor based on the increase in the gene expression of PU.1 and/or the decrease in the gene expression of MT induced by the DNA methylation of the MT gene promotor region in the gene-expressing cell.
8. The detection method for a drug effect of a DNA methylation inhibitor according to any one of 1 to 7 above, in which the gene-expressing cell is a hematological malignant cell.
9. The detection method for a drug effect of a DNA methylation inhibitor according to any one of 1 to 8 above, in which 5-azacytidine and/or 5-aza-2'-deoxycytidine are/is used as the DNA methylation inhibitor.
10. The detection method for a drug effect of a DNA methylation inhibitor according to 6 above, in which the gene-expressing cell is a hematological malignant cell, 5-aza-2'-deoxycytidine (5-azadc) is used as the DNA methylation inhibitor, and the DNA methylation inhibitor resistance of the hematological malignant cell to the 5-azadc is detected based on PU.1/GAPDH of less than 0.01 and/or MT-1A/GAPDH or MT-1G/GAPDH of more than 0.002.
11. The detection method for a drug effect of a DNA methylation inhibitor according to 7 above, in which the gene-expressing cell is a hematological malignant cell, 5-aza-2'-deoxycytidine (5-azadc) is used as the DNAmethylation inhibitor, and the high DNAmethylation inhibitor sensitivity of the hematological malignant cell to the 5-azadc is detected based on PU.1/GAPDH of more than 0.1 and/or MT-1A/GAPDH or MT-1G/GAPDH of less than 0.002.
12. The detection method for a drug effect of a DNA methylation inhibitor according to 2 above, in which the drug resistance or high drug sensitivity of the gene-expressing cell to the DNA methylation inhibitor is detected based on the DNA methylation ratio of the metallothionein (MT) gene promotor region.
13. The detection method for a drug effect of a DNA methylation inhibitor according to 6 or 12 above, in which the gene-expressing cell is a hematological malignant cell, 5-aza-2'-deoxycytidine (5-azadc) is used as the DNA methylation inhibitor, and the DNA methylation inhibitor resistance of the hematological malignant cell to the 5-azadc is detected based on a DNA methylation ratio of less than 20%.
14. The detection method for a drug effect of a DNA methylation inhibitor according to 7 or 12 above, in which the gene-expressing cell is a hematological malignant cell, 5-aza-2'-deoxycytidine (5-azadc) is used as the DNA methylation inhibitor, and the high DNA methylation inhibitor sensitivity of the hematological malignant cell to the 5-azadc is detected based on a DNA methylation ratio of more than 50%.

### Advantageous Effects of Invention

The present invention provides the detection method for a drug effect of a DNA methylation inhibitor, which serves as a marker for predicting a drug effect of the DNAmethylation inhibitor.
According to the present invention, therapeutic results are improved because the DNA methylation inhibitor can be applied to hematological malignant cells with myelodysplasia syndrome, acute myeloid leukemia (AML), and any other hematopoietic disease after the detection of drug sensitivity based on the gene expression of the transcription factor (PU.1) involved in neutrophil and monocyte lineage commitment, the gene expression of its target gene metallothionein (MT), and/or the methylation ratio of the MT gene promotor region.

### Brief Description of Drawings

[FIGS. 1] FIGS. 1 illustrate and show the establishment of PU.1 gene-knockdown cell lines (K562 PU.1 KD cells).
[FIGS. 2] FIGS. 2 illustrate increases in gene expression of an MT-1 family in the K562 PU.1 KD cells.
[FIGS. 3] FIGS. 3 illustrate and show the establishment of PU.1 gene-overexpressing cell lines (K562 PU.1 OE cells).
[FIGS. 4] FIGS. 4 illustrate decreases in gene expression of an MT-1 family in the K562 PU.1 OE cells.
[FIGS. 5] FIGS. 5 illustrate the results of MT-1G and show that an MT1 gene promotor region is demethylated in the PU.1 gene-knockdown cells.
[FIGS. 6] FIGS. 6 illustrate the results of MT-1G and show that the MT1 gene promotor region is methylated in the PU.1 gene-overexpressing cells.
[FIG. 7] FIG. 7 illustrates the comparisons of sensitivities (percentages of dead cells after drug administration) of the PU.1 gene-knockdown cells (PU 2-10 and PU 3-10) to 5-azadc with those of control cells (vec 5 and vec 6).
[FIG. 8] FIG. 8 illustrates the comparisons of sensitivities (percentages of dead cells after drug administration) of the PU.1 gene-overexpressing cells (A2 and H8) to 5-azadc with those of control cells (vec 1 and vec 2).

### Best Mode for Carrying Out the Invention

Blood cells develop from stem cells. The stem cells differentiate into various lineages, finally producing blood cells having various forms and functions. In such differentiation (branching), nuclear factors called transcription factors play important roles for the modulation of the expression of genes that specifically express the differentiated lineages.

Among such transcription factors, PU.1 plays an important role in neutrophil and monocyte lineage commitment. That is, analysis using mice reveals that acute myeloid leukemia (AML) develops by simply decreasing the expression of the transcription factor PU.1. As described above, a decrease in expression of the hematopoietic transcription factor PU.1 plays a central role in the development of AML, which is leukemia of the most frequent type in adult humans.

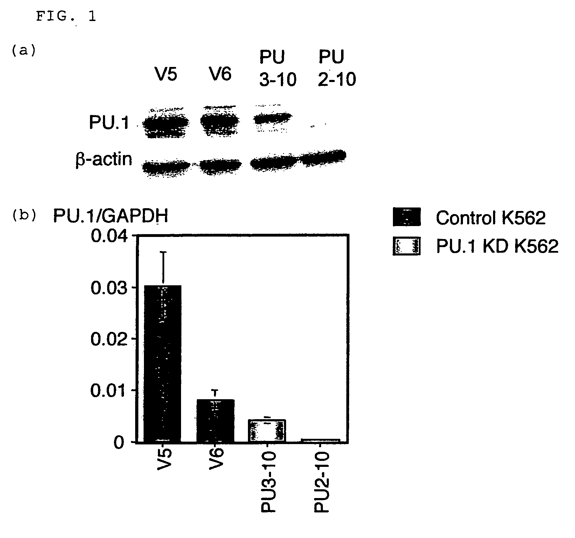

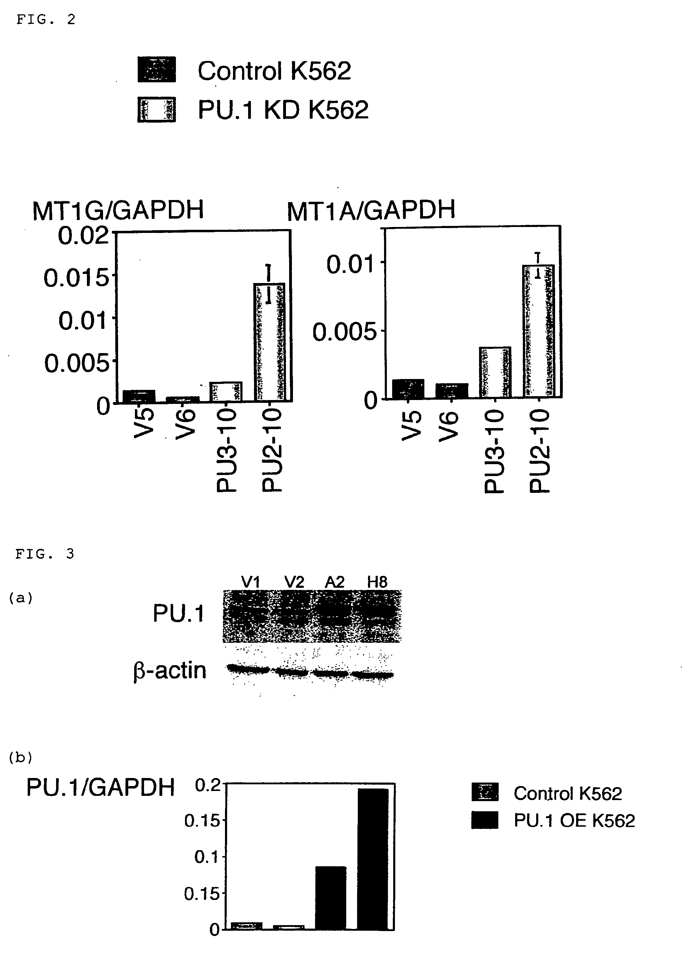

In order to clarify the pathogenesis and mechanism of AML, cell lines (K562 PU.1 KD cells) with decreased expression of only PU.1 were artificially prepared using a siRNA method that involves suppressing the expression of PU.1 in leukemia cells (K562 cells). Then, through comparisons with control cells, attempts were made on the identification of genes with varying levels of expression depending on decreases in expression of PU.1 using a microarray method that involves exhaustively analyzing gene expression. The results revealed that the expression of a group of metallothionein (MT) genes (such as MT-1A and MT-1G) was increased along with a decrease in expression of PU.1 (FIGS. 1 and FIGS. 2).

FIGS. 1 (a) and 1 (b) illustrate and show the results of establishment experiments of PU.1-knockdown lines (K562 PU.1 KD cells). That is, a PU.1 siRNA expression vector (Takara, Ohtsu, Japan) and its control vector (Takara) were introduced into K562 cells as leukemia cell lines in accordance with the method described in the document (Takahashi S et al. , British Journal of Haematology, 2005(130),p428-436). Specifically, in the method, electroporation was employed to introduce the expression vector and its control vector into leukemia cell lines (K562 cells). The introduced cells were cultured in an RPMI medium in the presence of 1 µg/mL puromycin to select cells that constitutively express PU.1 siRNAs and isolate the cells by a limiting dilution method. The isolated control cells (Control K562) (V5 and V6) and PU.1-knockdown cells (2-10 and 3-10) (K562 PU.1 KD cells) were used to prepare proteins based on a conventional method. The proteins were subjected to SDS-PAGE electrophoresis, then transferred to a membrane, and blotted with a PU.1 antibody and β-actin useful for the estimation of protein amounts (FIG. 1(a)).

Further, RNAs prepared from the above-mentioned cells based on a conventional method were used to synthesize cDNAs, and quantitative PCR (conditions: see Examples) was then performed. The expression amounts of PU.1 were corrected with the expression amounts of glyceraldehydes-3-phosphate dehydrogenase (GAPDH) as a housekeeping gene, and was illustrated as PU.1/GAPDH in FIG. 1 (b).
The results clarified that the expression of PU. 1 was decreased in the PU.1-knockdown cells (K562 PU.1 KD cells) (2-10 and 3-10) as compared to the control lines (V5 and V6).

FIGS. 2 illustrate that the expression of an MT-1 family gene is increased in cell lines with artificially decreased expression of only PU.1 (K562 PU.1 KD cells).
RNAs prepared from K562 PU.1 KD cells (PU 2-10 and PU 3-10) and their control cells (Control K562) (V5 and V6) based on a conventional method were used to synthesize cDNAs, and quantitative PCR was performed under conditions described in Examples later in order to investigate the expression of MT1A and MT1G. As a result, the expression amounts of MT1A and MT1G were corrected with the expression amounts of glyceraldehydes-3-phosphate dehydrogenase (GAPDH) as a housekeeping gene, and were illustrated as MT1A/GAPDH and MT1G/GAPDH in FIGS. 2.
The results revealed that the expression amount of any gene of MT1A and MT1G was increased in the K562 PU.1 KD cells.

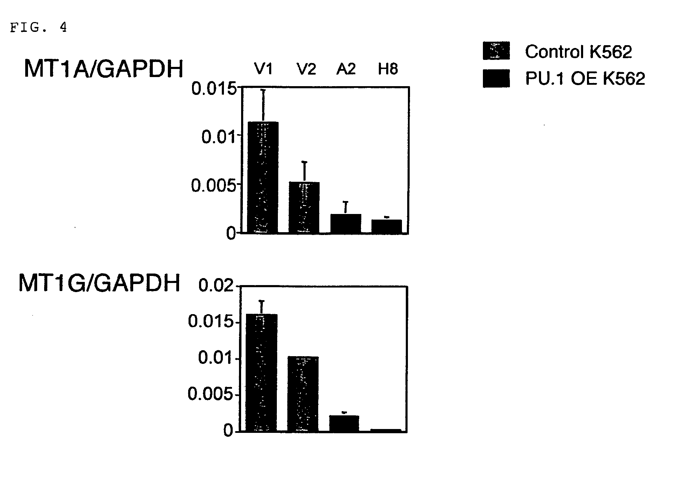

In contrast to the above, PU.1-overexpressing cells (K562 PU.1 OE cells) were artificially produced to investigate the expression of a PU. 1 gene. The results confirmed that the expression of an MT gene was decreased, revealing that such gene was a bona fide target gene (FIGS. 3 and FIGS. 4).

FIGS. 3 (a) and 3 (b) illustrate and show the results of establishment experiments of PU.1-overexpressing cell lines (K562 PU.1 OE cells). That is, the PU.1 expression vector described in the literature was used (Inomata et al., Leukemia Research 30 (2006) p659-664). The expression vector and its control vector pcDNA3.1 (Invitrogen, CA) were introduced into K562 cells as leukemia cell lines in accordance with the method described in literature (Takahashi S et al., British Journal of Haematology, 2005(130), p428-436). The introduced cells were cultured in an RPMI medium in the presence of 400 µg/mL Neomycin to select cells that constitutively express PU.1 and isolate the cells by a limiting dilution method. The isolated control cells (Control K562) (V1 and V2) and PU.1-overexpressing cells (K562 PU.1 OE cells) (A2 and H8) were used to prepare proteins based on a conventional method. The proteins were subjected to SDS-PAGE electrophoresis, then transferred to a membrane, and blotted with a PU.1 antibody and β-actin useful for the estimation of protein amounts (FIG. 3(a)).
Further, RNAs prepared from the above-mentioned cells based on a conventional method were used to synthesize cDNAs, and quantitative PCR (conditions: see Examples) was then performed under conditions described in Examples later. The results were corrected with the expression amounts of glyceraldehydes-3-phosphate dehydrogenase (GAPDH) as a housekeeping gene, and were illustrated as PU.1/GAPDH in FIG. 3(b).
The results clarified that the expression of PU. 1 was increased in the PU.1-overexpressing cells (K562 PU.1 OE cells) (A2 and H8) as compared to the control lines (Control K562) (V1 and V2).

FIGS. 4 illustrate that the expression of an MT-1 family gene is decreased in PU.1-overexpressing cells (K562 PU.1 OE cells).
RNAs prepared from K562 PU.1 OE cells (A2 and H8) and their control cells (Control K562) (V1 and V2) based on a conventional method were used to synthesize cDNAs, and quantitative PCR was performed under conditions described in Examples later in order to investigate the expression of MT1A and MT1G. The results were corrected with the expression amounts of glyceraldehydes-3-phosphate dehydrogenase (GAPDH) as a housekeeping gene, and were illustrated as MT1A/GAPDH and MT1G/GAPDH in FIGS. 4.
The results revealed that the expression amount of any gene of MT1A and MT1G was decreased in the K562 PU.1 OE cells.

In transcription in which mRNA is synthesized from DNA, it is known that transcription amounts are quite different depending on whether DNA is tightly or loosely wrapped around nuclear proteins, histones. Changes in structure of chromatin as a complex of nuclear DNA and proteins as described above are important for transcriptional regulation. Of those, more attention should be focused on a DNA methylation mechanism. The occurrence of DNA methylation suppresses the transcription.

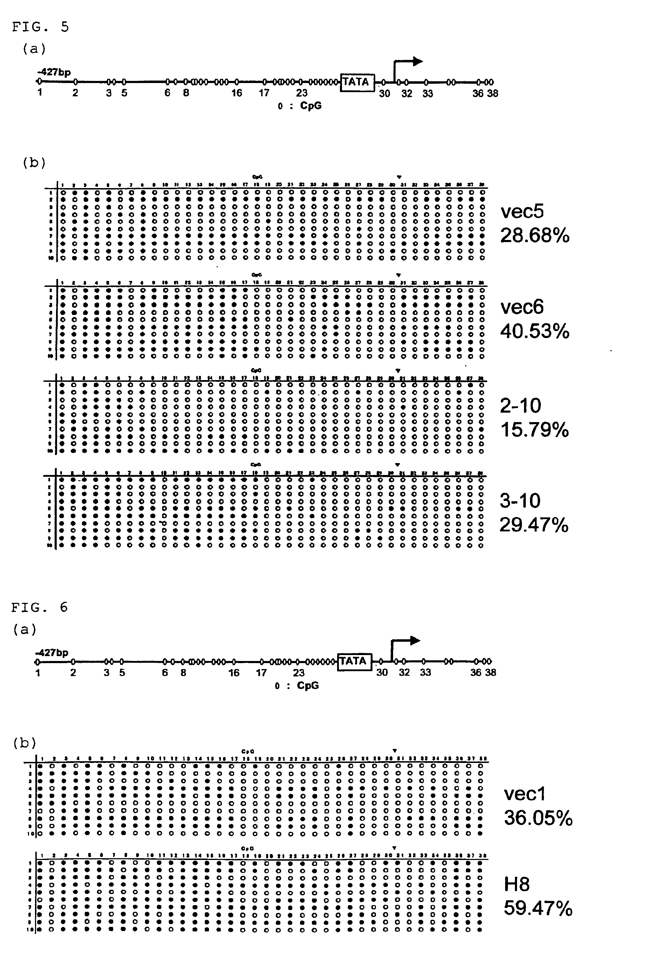

The inventor of the present invention discovered that the above-mentioned increase in expression of the MT gene due to the decrease in expression of the PU.1 gene was regulated by the DNA methylation mechanism, in other words, that a decrease in DNA methylation of the MT gene promotor region was involved in an increase in expression of the gene (FIGS. 5 and FIGS. 6). Such gene methylation is decreased in a plurality of gene promotor regions of the PU.1 gene-knockdown cells as well as the MT gene promotor region. Those results suggest that the DNA methylation inhibitor does not provide any sufficient drug effect in leukemia due to the decrease in expression of the PU.1 gene.

FIGS. 5 (a) and 5(b) illustrate the results of MT-1G and illustrate that the MT1 gene promotor region is demethylated in the PU.1 gene-knockdown cells.
The 5'-upstream region of the MT-1G gene is illustrated in the schematic diagram of FIG. 5(a). The arrow indicates the transcription start site reported in the literature (Huang et al., Int. J. Cancer: 104, p735-744 (2003)). The TATA box and CpG islands (open circles: o) were illustrated. The numbers represent the numbers of CpG islands from -427 bp upstream of the transcription start site analyzed in this study.
FIG. 5(b) illustrates the results of DNA methylation base sequence analysis of an MT-1G gene promoter. Genomic DNAs were prepared from cells illustrated in the figure, subj ected to bisulfite treatment, then amplified by PCR using primers described in Examples later, and cloned into a PGEMT easy vector (Promega, Madison, WI) to perform sequence analysis. Methylated CpG islands are represented by filled circles (•) and unmethylated CpG islands are represented by open circles (o).
The results revealed that the PU.1-knockdown cells (2-10: methylation ratio of 15.79% and 3-10: methylation ratio of 29.47%) showed a decreased methylation ratio as compared to the control cells (vec 5: methylation ratio of 28.68% and vec 6: methylation ratio of 40.53%).

FIGS. 6 (a) and 6 (b) illustrate the results of MT-1G and illustrate that the MT1 gene promotor region is methylated in the PU.1 gene-overexpressing cells.
The 5'-upstream region of the MT-1G gene is illustrated in the schematic diagram of FIG. 6(a) like FIG. 5(a) above.
FIG. 6(B) illustrates the results of DNA methylation base sequence analysis of an MT-1G gene promoter. Genomic DNAs were prepared from cells illustrated in the figure, subjected to bisulfite treatment, then amplified by PCR using primers described in Examples later, and cloned into a PGEMT easy vector (Promega, Madison, WI) to perform sequence analysis. Methylated CpG islands are represented by filled circles (•) and unmethylated CpG islands are represented by open circles (o).
The results revealed that the PU.1 gene-overexpressing cell (H8: methylation ratio of 59.47%) showed an increased methylation ratio as compared to the control cell (vec 1: methylation ratio of 36.05%).

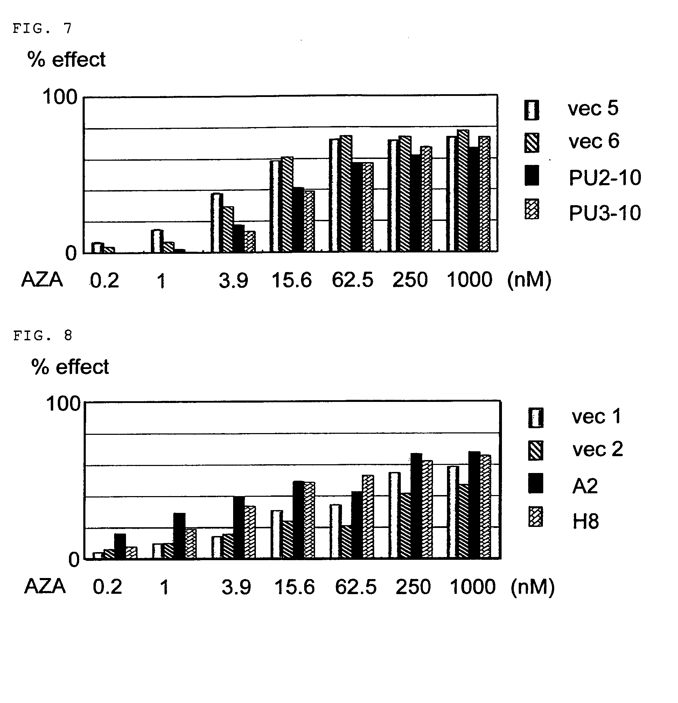

In view of the foregoing, with the use of the PU.1 gene-knockdown cells (K562 PU.1 KD cells) and the PU.1 gene-overexpressing cells (K562 PU.1 OE cells), 5-aza-2'-deoxycitidine (sometimes abbreviated as 5-azadc or AZA), which was a DNA methylation inhibitor and was a drug used practically in the clinical field, was administered to those cells. The results revealed that the PU.1 gene-knockdown cells, in which a DNA methylation activity was inhibited, were resistant and poorly sensitive to 5-azadc (FIG. 7), and on the contrary, the PU.1 gene-overexpressing cells were highly sensitive to 5-azadc (FIG. 8).

Two kinds of PU.1 gene-knockdown cells (PU 2-10 and PU 3-10) and control cells (vec 5 and vec 6) were compared for their sensitivities to 5-azadc (AZA) (% effect: percentages of dead cells after drug administration). In other words, the respective cells were seeded in a 96-well plate at a density of 2,000 cells/well, and 5-azadc (AZA) was added into the well at concentrations of 0.2, 1, 3. 9, 15. 6, 62. 5, 250, and 1,000 nM. Then, cell viability assays were performed after 96 hours. In the assays, 10 µL of a WST-8 reagent (Dojindo, Japan) were added to a well in which 100 µL of a cell culture medium had been loaded, culture was performed in a 5% CO₂ incubator at 37 °C for 2 hours, and the absorbance is then measured using a microplate reader at a wavelength of 490 nm, to thereby measure cell viability. FIG. 7 illustrates the results in terms of percentages of dead cells (% effect).
The results revealed that the PU.1 gene-knockdown cells (PU 2-10 and PU 3-10) showed low percentages of dead cells by about 20% as compared to the control cells (vec 5 and vec 6) and hence had drug resistance.

Two kinds of PU.1 gene-overexpressing cells (A2 and H8) and control cells (vec 1 and vec 2) were compared for their sensitivities (% effect: percentage of dead cells after drug administration) to 5-azadc (AZA). The same experiment as FIG. 7 above was performed. FIG. 8 shows the results. The results revealed that the PU.1 gene-overexpressing cells had high percentages of dead cells by about 20% as compared to the control cells and hence had high drug sensitivity.

In cancer, the aberrant methylation of a cancer suppressor gene promotor region was observed with high frequency. Based on the observation, the aberrant methylation has been estimated to be involved in the pathogenic mechanism of cancer. There is a growing awareness that a DNA methylation inhibitor, which has less adverse effects than an chemotherapeutic agent, exerts its effect on myelodysplasia syndrome recognized as a preleukemic medical condition. The DNA methylation inhibitor has started to be used for hematopoietic malignant diseases other than myelodysplasia syndrome.

An AML1 gene, which is expressed at the stage of stem cells and is an important transcription factor for hematopoiesis, yields an aberrant fusion product called AML1-ETO owing to aberration called reciprocal translocation in which chromosomes 8 and 21 are partially replaced in part of acute myeloid leukemia (AML). The aberrant fusion transcription factor is presumably involved in the pathology through the aberrant regulation of the expression of genes involved in the differentiation of hematopoietic cells. Several AML-specific fusion transcription factors including AML1-ETO are each said to have a high DNA methylation activity, and hence studies on the administration of the methylation inhibitor are being conducted in AML as well. However, the effects have not been made clear yet.

In other words, it is conceivable that the DNA methylation inhibitor does not simply provide remarkably effects on all AML cells. Indeed, also in the discovery of the inventor of the present invention in this study, the decrease in expression of the PU.1 transcription factor as an AML-simulating condition was found to lead to a decrease in intracellular methylation and drug resistance.
In contrast, an increase in expression of the PU.1 transcription factor was found to lead to an increase in intracellular methylation and an increase in drug sensitivity. In other words, according to the present invention, in hematopoietic diseases such as myelodysplasia syndrome and AML, studies on the expression of PU. 1 and the expression of its target gene MT were found to be usable for more effectively predicting and detecting an effect of the DNA methylation inhibitor on AML.

In other words, studies on (1) the expression amount of the transcription factor PU.1, (2) the expression amount of MT (e.g., MT-1A or MT-1G), and (3) the methylation of the MT (e.g., MT-1A or MT-1G) gene promotor region were found to serve as a clinically useful marker for predicting and detecting an effect of a DNA methylation inhibitor such as 5-azadc.

The results of Examples revealed that, when the level of PU.1/GAPDH was less than 0.01 (FIGS. 1) or when the level of MT-1A or G/GAPDH was more than 0.002 (FIGS. 2), the sensitivity to 5-azadc was low, whereas when the level of PU.1/GAPDH was more than 0.1 (FIGS. 3) or when the level of MT-1A or G/GAPDH was less than 0.002 (FIGS. 4), the sensitivity to 5-azadc was high. The results also revealed that, when the methylation ratio was less than 20%, there was resistance to 5-azadc (FIGS. 5), whereas when the methylation ratio was more than 50%, the sensitivity was high (FIGS. 6).

5-azadc is a drug that has attracted attention as the DNA methylation inhibitor but 5-azadc alone is not a drug that is expected to exert a sufficient therapeutic effect on AML. However, the present invention revealed that studies on the gene expression of PU. 1 orMT clarified to serve as its target gene, the DNAmethylation of the gene promotor region, and the like before drug administration allowed the prediction and detection of a drug effect. Treatments based on such information allow effective and specific treatments of hematological malignancies such as AML without relying solely on an chemotherapeutic agent, and as a result, therapeutic results are improved.

According to the present invention, there is provided a marker for predicting a drug effect with very high specificity based on the findings that hematological malignant cells with decreased expression of the PU.1 gene are resistant to the DNA methylation inhibitor such as 5-azadc, and hematological malignant cells with increased expression of the PU. 1 gene are highly sensitive to the DNA methylation inhibitor such as 5-azadc. Further, the methylation ratio of each of the MT genes such as MT-1A and MT-1G has a correlation with the expression of the PU.1 gene, and hence the detection of the methylation ratio of a group of those genes is also useful as a marker for predicting a drug effect of the DNA methylation inhibitor such as 5-azadc.

According to the present invention, a drug effect of a DNA methylation inhibitor including not only 5-aza-2'-deoxycytidine(5-azadc) but also 5-azacytidine or a mixture of 5-azacytidine and 5-azadc can be effectively predicted and detected.

### Examples

Hereinafter, the present invention is described in more detail with reference to examples, however, the present invention is not limited to these examples.

### Example

### [Studies on expression amounts of transcription factor PU.1 and target gene MT-1]

Monocytes were separated from a specimen having a high percentage of blast cells and being derived from bone marrow blood or peripheral blood of a patient who gave informed consent based on a conventional method by a density-gradient centrifugation method using a Ficoll Hypaque (GE Healthcare Bio-Sciences,Uppsala,Sweden). Total RNAs were prepared from the separated monocytes based on a conventional method using an Isogen (NIPPON GENE). The resultant RNAs were used to synthesize cDNAs in accordance with a conventional method using a SuperScript First-Strand system (Invitrogen, Carlsbad, CA).

Primers used for quantitative PCRare as describedbelow:
MT-1G forward; 5'-CTTCTCGCTTGGGAACTCTA-3' (SEQ ID NO: 1);
reverse; 5'-AGGGGTCAAGATTGTAGCAAA-3' (SEQ ID NO: 2);
MT-1A forward; 5'-CTCGAAATGGACCCCAACT-3' (SEQ ID NO: 3);
reverse; 5'-ATATCTTCGAGCAGGGCTGTC-3' (SEQ ID NO: 4);
PU.1 forward; 5'-GTGCCCTATGACAACGGATCT-3' (SEQ ID NO: 5); and
reverse; 5'-GAAGCTCTCGAACTCGCTGT-3' (SEQ ID NO: 6).

With the use of those primers, quantitative PCR was performed using an Opticon mini real-time PCR instrument (Bio-Rad, Hercules, CA) with a Quantitect SYBR green PCR reagent (Qiagen, Miami, FL) in accordance with the manufacturer's instructions, to thereby detect the expression amounts. Further, the expression amounts of all gene products were each calculated by being corrected with the expression amounts of GAPDH serving as a housekeeping gene described below.
GAPDH forward; 5'-GAAGGTGAAGGTCGGAGT-3' (SEQ ID NO: 7); and
reverse; 5'-GAAGATGGTGATGGGATTTC-3' (SEQ ID NO: 8).

PCR conditions are as described below:
PU.1 : step 1, 95°C for 15 minutes;
step 2, 95°C for 15 seconds;
step 3, 60°C for 1 minute; and
steps 2 and 3 are repeated 35 times.
MT-1A, G, and GAPDH:
step 1, 95°C for 15 minutes;
step 2, 95°C for 30 seconds;
step 3, 55°C for 30 seconds;
step 4, 72°C for 30 seconds; and
steps 2 to 4 are repeated 35 times.

The number of copies required for the detection of expression amounts was calculated in accordance with the previously reported method (Takahashi S et al., Leukemia Research, 2005 (29), p893-899). It should be noted that the method specifically involves cloning DNAs amplified with the above-mentioned primers into a PGEMT easy vector (Promega, Madison, WI) to prepare plasmid DNAs specific for the genes, then calculating the number of copies based on the mass of the prepared plasmid DNAs to prepare dilution series of plasmid, performing quantitative PCR in conjunction with cDNAs to be measured, and calculating the number of copies required for the detection of expression amounts from a calibration curve from the dilution series.

The results in the prepared culture cells revealed that the cells were resistant and poorly sensitive to 5-azadc in the case of a level of PU.1/GAPDH of less than 0.01 (FIGS. 1) or a level of MT-1A or G/GAPDH of more than 0.002 (FIGS. 2), the level being calculated by the above-mentioned method, and the cells were highly sensitive to 5-azadc in the case of a level of PU.1/GAPDH of more than 0.1 (FIGS. 3) or a level of MT-1A or G/GAPDH of less than 0.002 (FIGS. 4), the level being calculated by the above-mentioned method.

### [Studies on methylation of MT-1 gene promotor region]

Monocytes were separated from a specimen having a high percentage of blast cells and being derived from bone marrow blood or peripheral blood of a patient who gave informed consent based on a conventional method by a density-gradient centrifugation method using a Ficoll Hypaque (GEHealthcareBio-Sciences, Uppsala, Sweden). Genomic DNAs were prepared from the separated monocytes using a Qiagen Blood & Cell Culture DNA mini kit (QIAGEN). When the DNAs are subjected to bisulfite treatment based on a conventional method using an EZ DNA Methylation-Gold kit (Zymo Research, Orange, CA), methylated cytosine is not converted, and only unmethylated cytosine is converted to uracil. The resultant DNAs were amplified with the following primers and cloned into a PGEMT easy vector (Promega, Madison, WI).
MT-1G forward; 5'-TTTGGTAGATTTAGAAAGTGGAGTATAAGA-3' (SEQ ID NO: 9);
reverse; 5'-CTCAAACCCAAAAACACTCTCTATAATATC-3' (SEQ ID NO: 10);
MT-1A forward; 5'-GGGATAGGAGTAGGAGGTTGTGGTTGTATT-3' (SEQ ID NO: 11); and
reverse; 5'-ACACCTCTACTCCTAA.AAACATCTATCCTATA-3' (SEQ ID NO: 12).

After having been cloned into the vector, the DNAs were subjected to sequence analysis based on a conventional method using an ABI Prism DNA sequencer 3130 (Applied Biosystems) with a Big Dye Sequencing kit (Applied Biosystems, Foster City, CA) to examine the conversion of cytosine to thymine, to thereby calculate a methylation ratio. As a sequence reaction, uracil is expressed as thymine, and the sequence expression of cytosine and thymine (uracil) to be generated after bisulfite treatment varies depending on the presence or absence of methylation.

The results of FIGS. 5 and FIGS. 6 revealed that the cells were resistant and poorly sensitive to 5-azadc in the case of a methylation ratio of less than 20% (FIGS. 5) and the cells were highly sensitive to 5-azadc in the case of a methylation ratio of more than 50% (FIGS. 6).

### Industrial Applicability

The present invention provides the detection method for a drug effect for effectively applying a methylation inhibitor serving as a molecular target drug to hematological malignant cells with myelodysplasia syndrome, acute myeloid leukemia (AML), and any other hematopoietic disease.
According to the present invention, hematological malignancies can be treated effectively and specifically after the detection of sensitivity to a drug (methylation inhibitor) based on the gene expression of a transcription factor (PU.1) involved in neutrophil and monocyte lineage commitment, the gene expression of its target gene metallothionein (MT), and/or the methylation ratio of the MT gene promotor region.

## Claims

1. A detection method for a drug effect of a DNA methylation inhibitor, including detecting, based on the gene expression of a transcription factor (PU.1) involved in neutrophil or monocyte lineage commitment and/or the gene expression of metallothionein (MT), the drug sensitivity of a gene-expressing cell to the DNA methylation inhibitor.

2. A detection method for a drug effect of a DNA methylation inhibitor, the method including detecting, based on DNA methylation in a metallothionein (MT) gene promotor region, the drug sensitivity of a gene-expressing cell to the DNA methylation inhibitor.

3. The detection method for a drug effect of a DNA methylation inhibitor according to claim 1, in which the drug sensitivity of the gene-expressing cell to the DNAmethylation inhibitor is detected based on the fact that the gene expression of a transcription factor (PU.1) involved in neutrophil or monocyte lineage commitment and/or the gene expression of metallothionein (MT) are/is regulated by the DNA methylation of a metallothionein (MT) gene promotor region in the gene-expressing cell.

4. The detection method for a drug effect of a DNA methylation inhibitor according to claim 1 or 3, in which the gene-expressing cell is detected to be resistant to the DNA methylation inhibitor based on a decrease in the gene expression of PU.1 and/or an increase in the gene expression of MT.

5. The detection method for a drug effect of a DNAmethylation inhibitor according to claim 1 or 3, in which the gene-expressing cell is detected to be highly sensitive to the DNA methylation inhibitor based on an increase in the gene expression of PU.1 and/or a decrease in the gene expression of MT.

6. The detection method for a drug effect of a DNA methylation inhibitor according to claim 4, in which the gene-expressing cell is detected to be resistant to the DNA methylation inhibitor based on the decrease in the gene expression of PU.1 and/or the increase in the gene expression of MT induced by the DNA demethylation of the MT gene promotor region in the gene-expressing cell.

7. The detection method for a drug effect of a DNA methylation inhibitor according to claim 5, in which the gene-expressing cell is detected to be highly sensitive to the DNA methylation inhibitor based on the increase in the gene expression of PU.1 and/or the decrease in the gene expression of MT induced by the DNA methylation of the MT gene promotor region in the gene-expressing cell.

8. The detection method for a drug effect of a DNA methylation inhibitor according to any one of claims 1 to 7, in which the gene-expressing cell is a hematological malignant cell.

9. The detection method for a drug effect of a DNAmethylation inhibitor according to any one of claims 1 to 8, in which 5-azacytidine and/or 5-aza-2'-deoxycytidine are/is used as the DNA methylation inhibitor.

10. The detectionmethod for a drug effect of a DNAmethylation inhibitor according to claim 6, in which the gene-expressing cell is a hematological malignant cell, 5-aza-2'-deoxycytidine (5-azadc) is used as the DNA methylation inhibitor, and the DNA methylation inhibitor resistance of the hematological malignant cell to the 5-azadc is detected based on PU.1/GAPDH of less than 0.01 and/or MT-1A/GAPDH or MT-1G/GAPDH of more than 0.002.

11. The detectionmethod for a drug effect of a DNAmethylation inhibitor according to claim 7, in which the gene-expressing cell is a hematological malignant cell, 5-aza-2'-deoxycytidine (5-azadc) is used as the DNAmethylation inhibitor, and the high DNAmethylation inhibitor sensitivity of the hematological malignant cell to the 5-azadc is detected based on PU.1/GAPDH of more than 0.1 and/or MT-1A/GAPDH or MT-1G/GAPDH of less than 0.002.

12. The detection method for a drug effect of a DNAmethylation inhibitor according to claim 2, in which the drug resistance or high drug sensitivity of the gene-expressing cell to the DNA methylation inhibitor is detected based on the DNA methylation ratio of the metallothionein (MT) gene promotor region.

13. The detection method for a drug effect of a DNAmethylation inhibitor according to claim 6 or 12, in which the gene-expressing cell is a hematological malignant cell, 5-aza-2'-deoxycytidine (5-azadc) is used as the DNA methylation inhibitor, and the DNA methylation inhibitor resistance of the hematological malignant cell to the 5-azadc is detected based on a DNA methylation ratio of less than 20%.

14. The detection method for a drug effect of a DNAmethylation inhibitor according to claim 7 or 12, in which the gene-expressing cell is a hematological malignant cell, 5-aza-2'-deoxycytidine (5-azadc) is used as the DNA methylation inhibitor, and the high DNA methylation inhibitor sensitivity of the hematological malignant cell to the 5-azadc is detected based on a DNA methylation ratio of more than 50%.
